# EUROPEAN PATENT APPLICATION

(11) **EP 4 368 629 A1**
(43) Date of publication of application: **15.05.2024**
(21) Application number: 22837707.3
(22) Date of filing: 06.07.2022
(51) Int. Cl.: C07K 7/06, A61K 6/80, A61K 38/10, A61P 1/02, C07K 7/08

(54) **PEPTIDE AND COMPOSITION**

(30) Priority: 07.07.2021 JP 2021112587
(71) Applicant: OSAKA UNIVERSITY, Suita-shi Osaka 565-0871 (JP)
(72) Inventor: TAKAHASHI, Yusuke, Suita-shi, Osaka 565-0871 (JP); WATANABE, Masakatsu, Suita-shi, Osaka 565-0871 (JP); OKAMOTO, Motoki, Suita-shi, Osaka 565-0871 (JP); HAYASHI, Mikako, Suita-shi, Osaka 565-0871 (JP); KANIE, Kei, Nagoya-shi, Aichi 464-8601 (JP); KATO, Ryuji, Nagoya-shi, Aichi 464-8601 (JP); NARITA, Yuji, Nagoya-shi, Aichi 464-8601 (JP); OGATA, Aika, Nagoya-shi, Aichi 464-8601 (JP)
(74) Representative: Marks & Clerk LLP
(86) International application number: PCT/JP2022/026856
(87) International publication number: WO 2023/282293

(57) **Abstract**

The present invention addresses the problem of providing a peptide or a composition which can promote the healing of a wound at a dentin-pulp complex.

The problem can be solved by a peptide that comprises an amino acid sequence comprising Lys-Leu-Leu-Glu-Thr-Glu-Cys-Pro-Gln (SEQ ID NO: 28), an amino acid sequence comprising Asn-Thr-Asp-Gly-Ala-Val-Asn-Phe-Gln (SEQ ID NO: 32), an amino acid sequence comprising Glu-Leu-Val-Arg-Lys-Asp-Leu-Gln-Asn (SEQ ID NO: 47), an amino acid sequence comprising Asn-Ala-Asp-Lys-Gln-Leu-Ser-Phe-Glu (SEQ ID NO: 51), an amino acid sequence having such a structure that 1 to 3 amino acid residues in the amino acid sequence represented by SEQ ID NO: 28, SEQ ID NO: 32, SEQ ID NO: 47 or SEQ ID NO: 51 are substituted or deleted, or an amino acid sequence having such a structure that 1 to 3 amino acid residues are inserted into the amino acid sequence represented by SEQ ID NO: 28, SEQ ID NO: 32, SEQ ID NO: 47 or SEQ ID NO: 51, the peptide having a length of 9 to 15 amino acid residues.

## Description

### Technical Field

The present invention relates to a peptide and a composition which promote formation of dentin.

### Background Art

Dentin matrix components (DMCs), organic components of dentin, which account for about 20 % of the dentin, are proteins secreted by odontoblasts during the development of primary dentin.

In a series of previous studies investigating a pulp wound healing mechanism, it has been reported that a DMC degradation product generated by degradation with Matrix Metalloproteinase (MMP) 20 activate migration, differentiation, and mineralization abilities of pulp cells in vitro. In addition, the DMC degradation product induced tertiary dentin that completely covers the surface of exposed pulp in a direct pulp capping experiment in a rat in vivo, and the tertiary dentin also had a tubular structure, suggesting that the DMC degradation product generated by the MMP20 promote wound healing of dentin-pulp complex. Furthermore, it has been reported that Protein S100-A7 (S100-A7) and Protein S 100-A8 (S 100-A8), which were identified by proteomic analysis of the DMC degradation product generated by the action of the MMP20, activates proliferation, differentiation, and mineralization abilities of the pulp cells in vitro, and induces the tertiary dentin that completely covers the surface of the exposed pulp in the direct pulp capping experiment in the rat in vivo (Non Patent Literature 1), and it is believed that the S 100-A7 and the S 100-A8 promote the wound healing of the dentin-pulp complex.

### Citation List

### Non Patent Literature

Non Patent Literature 1: Komichi S, Takahashi Y, Okamoto M, Ali M, Watanabe M, Huang HL, Nakai T, Cooper P, Hayashi M: Protein S100-A7 derived from digested dentin is a critical molecule for dentin pulp regeneration. Cells 2019, 8(9): 19.

### Summary of Invention

### Technical Problem

The present invention addresses a problem of providing a peptide or a composition which can promote the wound healing of the dentin-pulp complex.

### Solution to Problem

As a result of diligent research, the present inventors have found that peptides derived from S100-A8 and S100-A9 of Protein S100 family have an ability to promote formation of dentin.

The present invention has been achieved based on the above finding, and includes the following aspects.
Item 1. A peptide comprising
   an amino acid sequence comprising Lys-Leu-Leu-Glu-Thr-Glu-Cys-Pro-Gln (SEQ ID NO: 28);
   an amino acid sequence comprising Asn-Thr-Asp-Gly-Ala-Val-Asn-Phe-Gln (SEQ ID NO: 32);
   an amino acid sequence comprising Glu-Leu-Val-Arg-Lys-Asp-Leu-Gln-Asn (SEQ ID NO: 47);
   an amino acid sequence comprising Asn-Ala-Asp-Lys-Gln-Leu-Ser-Phe-Glu (SEQ ID NO: 51);
   an amino acid sequence having such a structure that 1 to 3 amino acid residues in the amino acid sequence represented by SEQ ID NO: 28, SEQ ID NO: 32, SEQ ID NO: 47 or SEQ ID NO: 51 are substituted or deleted; or
   an amino acid sequence having such a structure that 1 to 3 amino acid residues are inserted into the amino acid sequence represented by SEQ ID NO: 28, SEQ ID NO: 32, SEQ ID NO: 47 or SEQ ID NO: 51,
   the peptide having a length of 9 to 15 amino acid residues.
Item 2. The peptide according to item 1, wherein the peptide comprises the amino acid sequence comprising
   Lys-Leu-Leu-Glu-Thr-Glu-Cys-Pro-Gln (SEQ ID NO: 28),
   Lys-Leu-Leu-Glu-Thr-Glu-Cys-Pro-Gln-Tyr (SEQ ID NO: 100), or
   Lys-Leu-Leu-Glu-Thr-Glu-Cys-Pro-Gln-Tyr-Ile (SEQ ID NO: 101).
Item 3. A composition comprising the peptide according to item 1 or 2.
Item 4. The composition according to item 3, wherein the composition is used to promote the formation of dentin.
Item 5. The composition according to item 3 or 4, wherein the composition is for filling a tooth cavity or for promoting pulp wound healing.

### Advantageous Effects of Invention

According to the present invention, a peptide or a composition capable of promoting wound healing of a dentin-pulp complex can be provided.

### Brief Description of Drawings

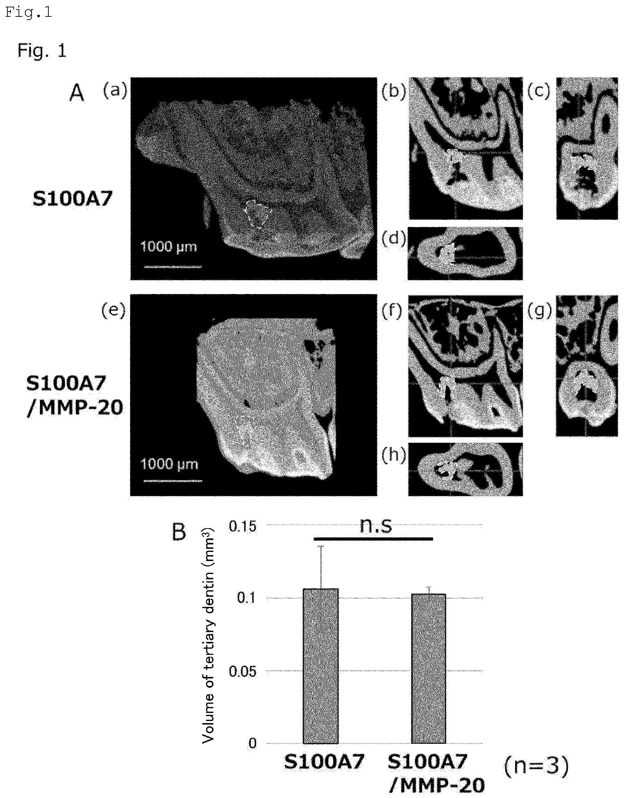

FIG. 1A shows a part of a three-dimensional constructed image (a, e), and planar images of a sagittal section (b, f), a frontal section (c, g), and a horizontal section (d, h) by micro-CT image analysis of a rat molar sample subjected to a direct pulp capping experiment using S100-A7 and a rat molar sample subjected to the direct pulp capping experiment using the S100-A7 reacted with MMP20. Tertiary dentin is indicated by the dashed line. FIG. 1B shows volume quantification results of the tertiary dentin (Student's t-test, p > 0.05).

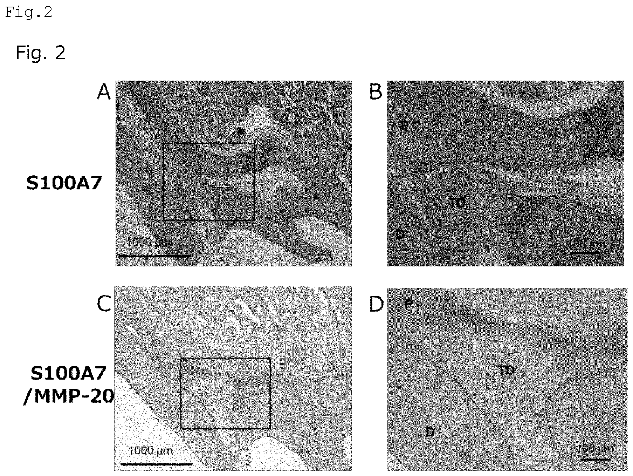

FIG. 2A shows an image of an HE-stained rat molar sample subjected to the direct pulp capping experiment using the S100-A7. FIG. 2B shows a magnified image of the boxed area in FIG. 2A. FIG. 2C shows an image of an HE-stained rat molar sample subjected to the direct pulp capping experiment using the S100-A7 reacted with the MMP20. FIG. 2D shows a magnified image of the boxed area in FIG. 2C. Dotted lines indicate the boundary between the tertiary dentin and primary dentin. Reference sign P indicates pulp, reference sign D indicates dentin, and reference sign TD indicates the tertiary dentin.

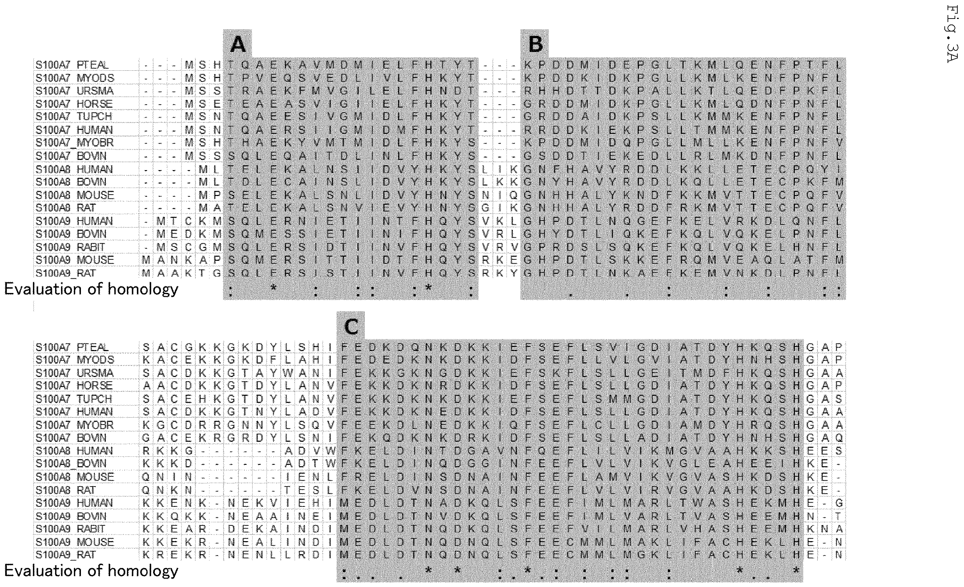

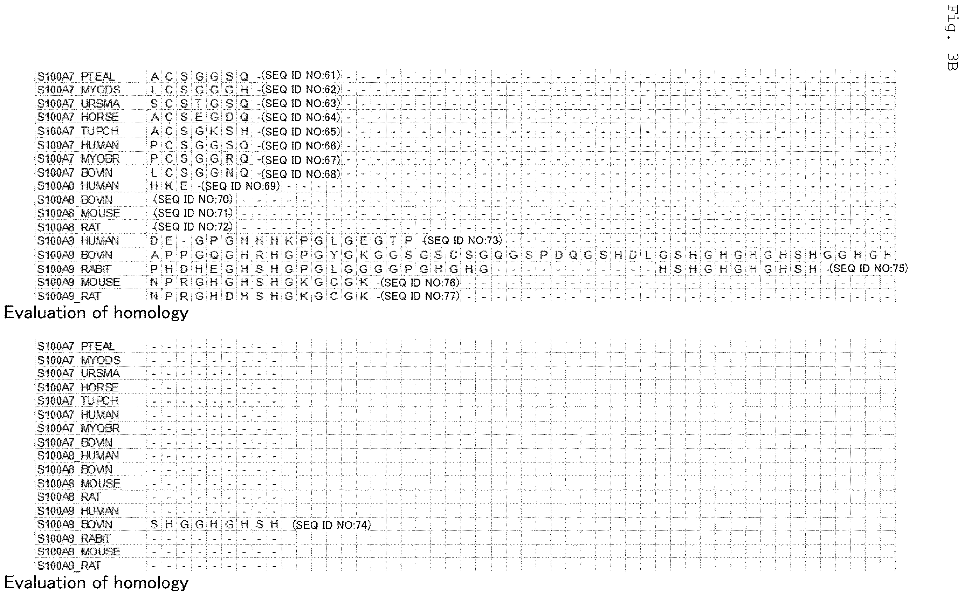

FIG. 3 shows the result of sequence alignment performed on the amino acid sequences of Protein S100 family. Sites where amino acids are identical are indicated by "*", sites with a homology score higher than 0.5 by CLUSTAL-W (sites with high homology) are indicated by ":", and sites with a homology score lower than 0.5 by CLUSTAL-W (sites with low homology) are indicated by ".", respectively, which are shown below the amino acid sequence. The sites with high homology are shaded.

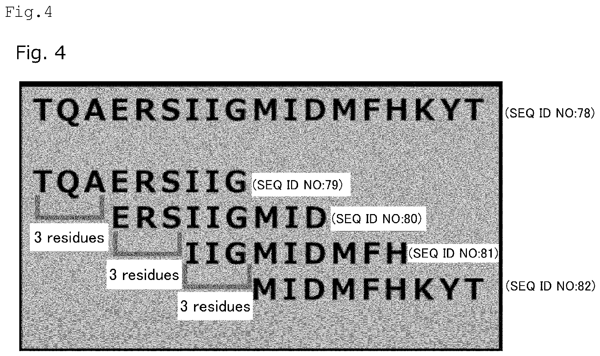

FIG. 4 shows an example of a design of a candidate peptide using S100-A7 HUMAN of A region shown in FIG. 3 as an example.

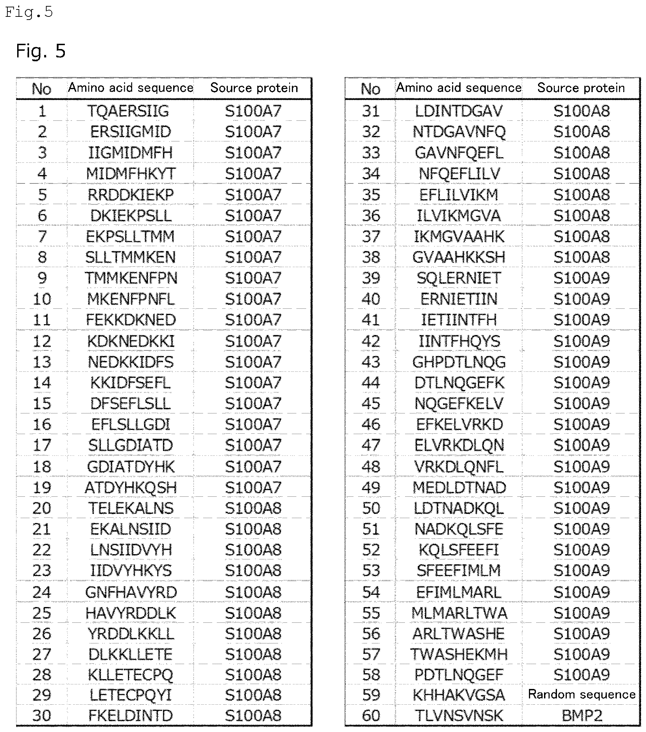

FIG. 5 shows a list of the candidate peptides.

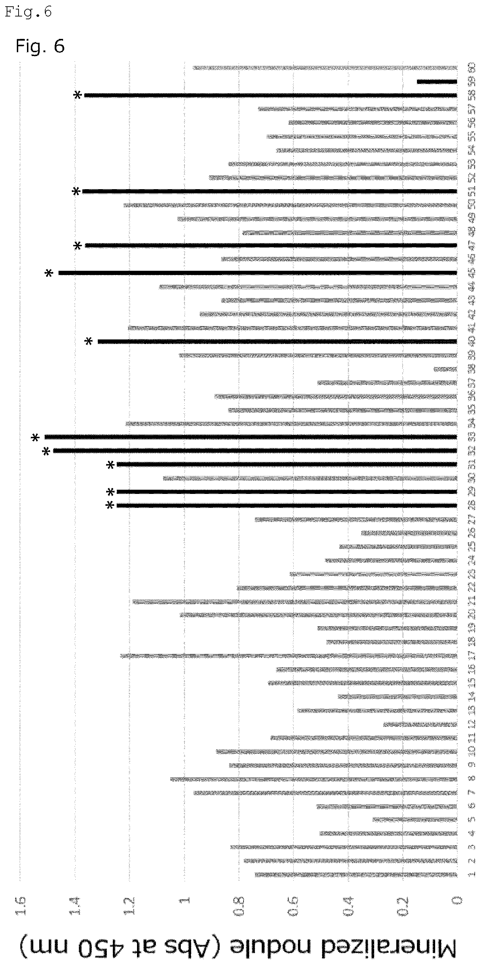

FIG. 6 shows mineralization ability of hDPSCs in the presence of each peptide for 58 candidate peptides. The "*" indicates the top 10 peptides that had higher mineralization ability.

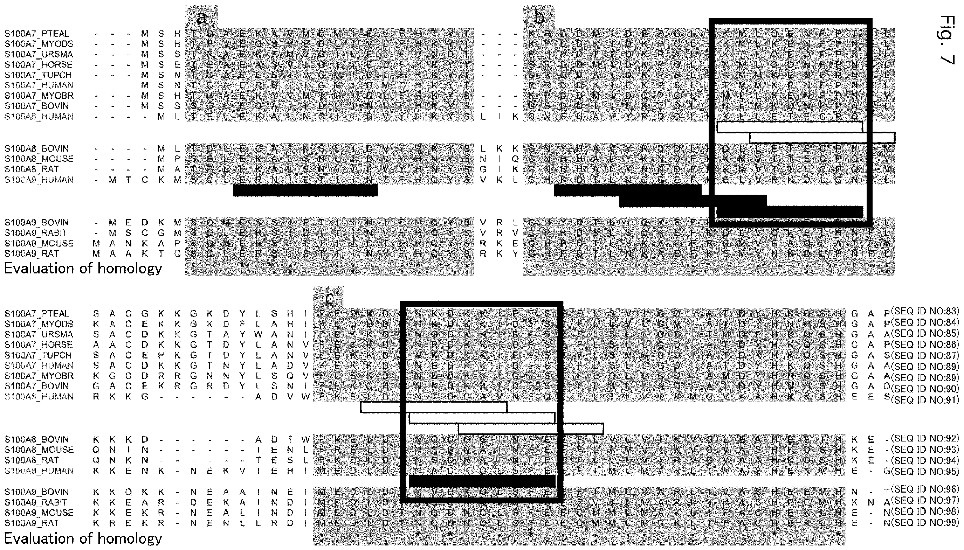

FIG. 7 shows the 10 sequence alignments marked with "*" in FIG. 6 compared to the results of peptide selection regions. Black boxed regions indicate overlapping regions of peptides that promoted mineralization of the hDPSCs. Peptides derived from the S100-A8 are indicated by white bars, and candidate peptides derived from the S100-A9 are indicated by black bars, at the bottom of the sequences.

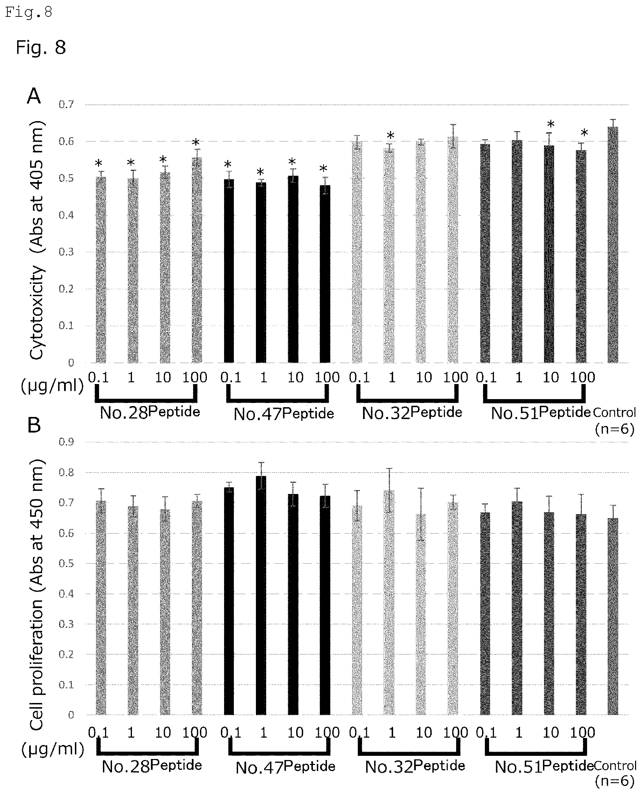

FIG. 8A shows cytotoxicity of Protein A100 family-derived peptides (No. 28, No. 32, No. 47, and No. 51) against hDPSCs. Samples with significant differences from the controls are indicated by * (One way ANOVA, Tukey's test, p < 0.05). FIG. 8B shows effects of each peptide on cell proliferative capacity.

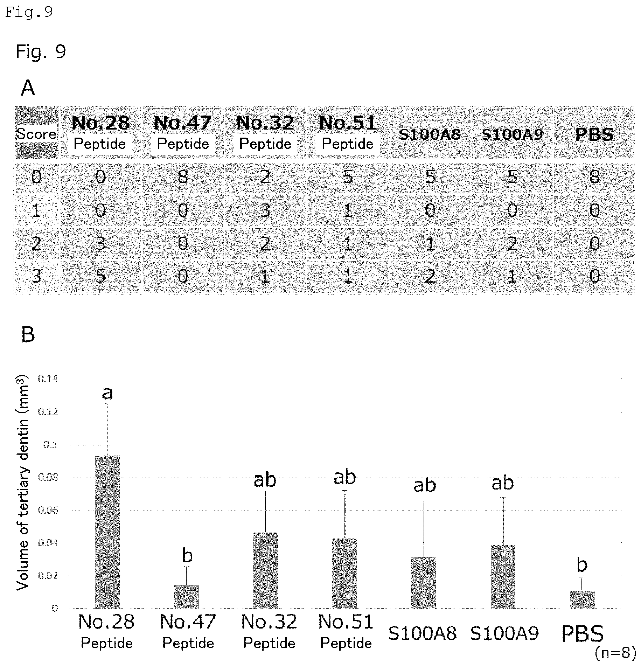

FIG. 9A shows the results of scoring the tertiary dentin formed after the pulp capping. FIG. 9B shows the volume of tertiary dentin formed after the pulp capping.

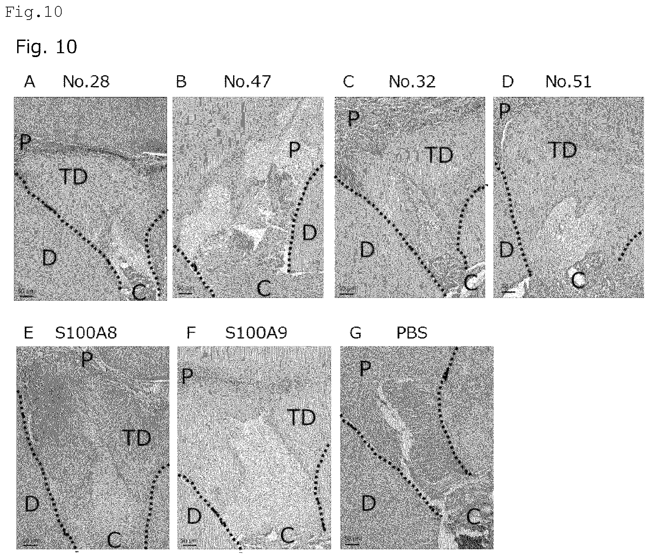

FIG. 10 shows images of HE stained-representative samples at 4 weeks after the direct pulp capping experiment using the Protein A100 family-derived peptides. FIG. 10A shows the image of the HE stained-sample treated with the No. 28 peptide. FIG. 10B shows the image of the HE stained-sample treated with the No. 47 peptide. FIG. 10C shows the image of the HE stained-sample treated with the No. 32 peptide. FIG. 10D shows the image of the HE stained-sample treated with the No. 51 peptide. FIG. 10E shows the image of the HE stained-sample treated with the S100-A8. FIG. 10F shows the image of the HE stained-sample treated with the S100-A9. FIG. 10G shows the image of the HE stained-sample treated with PBS. A dotted line indicates a boundary between the tertiary dentin and the primary dentin. Reference sign P indicates the pulp, reference sign D indicates the dentin, reference sign TD indicates the tertiary dentin, and reference sign C indicates an experimentally formed cavity.

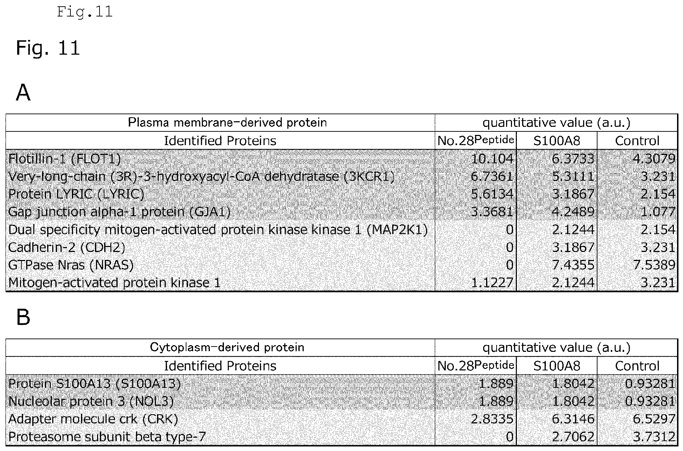

FIG. 11 shows characteristic proteins identified by LC-MS/MS analysis of proteins expressed when the Protein S100-A8 family-derived peptide (No. 28 peptide) or the S100-A8 was applied to the hDPSCs. FIG. 11A shows plasma membrane-derived proteins. Figure 11B shows cytoplasmic proteins. Quantitative value indicates a value (a.u.) measured for each sample.

### Description of Embodiments

### 1. Peptide

One embodiment of the present invention relates to a peptide.

The peptide is derived from Protein S100-A8 [UniProtKB-P05109 (S10A8_HUMAN)] or Protein S100-A9 [UniProtKB - P06702 (S10A9_HUMAN)]. Protein S100 family, to which Proptein S100-A7, the Proptein S100-A8, and the Proptein S100-A9 belong, is a group of low molecular weight proteins with molecular weights ranging from 8 to 14 kDa that are expressed only in vertebrates. The Protein S100 family members exist intracellularly and function by binding to nucleic acids and transcription factors, or may be released extracellularly upon cell damage or cell death as one of Damage-Associated Molecular Patterns (DAMPs).

In the present description, an amino acid sequence of a peptide shall be written from the N-terminal side to the C-terminal side. The representation and physical properties of the amino acid sequence shall be in accordance with Table 1 below.

**Table 1**

| Amino acid name | One letter code | Three letter code | Physical property at pH 7 | Hydropathy |
|---|---|---|---|---|
| Alanine | A | Ala | Nonpolar | Hydrophobic |
| Cysteine | C | Cys | Polar | Moderate |
| Aspartic Acid | D | Asp | Acidic | Hydrophilic |
| Glutamic Acid | E | Glu | Acidic | Hydrophilic |
| Phenylalanine | F | Phe | Nonpolar | Hydrophobic |
| Glycine | G | Gly | Nonpolar | Moderate |
| Histidine | H | His | Basic | Hydrophilic |
| Isoleucine | I | Ile | Nonpolar | Hydrophobic |
| Lysine | K | Lys | Basic | Hydrophilic |
| Leucine | L | Leu | Nonpolar | Hydrophobic |
| Methionine | M | Met | Nonpolar | Hydrophobic |
| Asparagine | N | Asn | Polar | Hydrophilic |
| Proline | P | Pro | Nonpolar | Hydrophobic |
| Glutamine | Q | Gln | Polar | Hydrophilic |
| Arginine | R | Arg | Basic | Hydrophilic |
| Serine | S | Ser | Polar | Hydrophilic |
| Threonine | T | Thr | Polar | Hydrophilic |
| Valine | V | Val | Nonpolar | Hydrophobic |
| Tryptophan | W | Trp | Nonpolar | Hydrophobic |
| Tyrosine | Y | Tyr | Nonpolar | Hydrophobic |

Amino acids include both natural and artificial amino acids.

As used herein, "residue" of various amino acids is a constituent unit of the amino acids constituting the peptide, and derived from the amino acids by removing a hydrogen atom for an amino group of a main chain and/or removing an -OH for a carboxyl group of the main chain.

In addition, "having" as used herein may include both concepts of "comprising" and "consisting of'.

A method for synthesizing the peptide is well known. For example, tert-butoxycarbonyl (Boc) solid-phase synthesis or 9-fluorenylmethyloxycarbonyl (Fmoc) solid-phase synthesis may be used. When the total length of the peptide main chain exceeds 100 amino acid residues, the peptide may be synthesized as a recombinant peptide using Escherichia coli, insect cells, mammalian cells, yeast, silkworm, or the like.

The peptide may also include those whose C-terminus is a carboxyl group (-COOH), carboxylate (-COO-), or ester (-COOR), etc.

The peptide comprises an amino acid sequence comprising Lys-Leu-Leu-Glu-Thr-Glu-Cys-Pro-Gln (SEQ ID NO: 28), an amino acid sequence comprising Asn-Thr-Asp-Gly-Ala-Val-Asn-Phe-Gln (SEQ ID NO: 32), an amino acid sequence comprising Glu-Leu-Val-Arg-Lys-Asp-Leu-Gln-Asn (SEQ ID NO: 47), or an amino acid sequence comprising Asn-Ala-Asp-Lys-Gln-Leu-Ser-Phe-Glu (SEQ ID NO: 51).

The length of said peptide is about 9 to 15 amino acid residues, preferably about 9 to 13 amino acid residues, more preferably about 9 to 10 amino acid residues.

The peptide comprises an amino acid sequence in which one to three amino acid residues, or one or two amino acid residues, are substituted or deleted in the amino acid sequence represented by SEQ ID NO: 1. A substitution of residues is preferably performed, for example, between amino acid residues having a similar degree of hydrophilicity.

The peptide may comprise an amino acid sequence in which one to three amino acid residues, or one or two amino acid residues, are inserted into the amino acid sequence represented by SEQ ID NO: 1.

The substitution, deletion, or insertion of two or three amino acid residues may occur as a contiguous amino acid sequence or as a discontiguous amino acid sequence.

Said peptide preferably comprises the amino acid sequence comprising Lys-Leu-Leu-Glu-Thr-Glu-Cys-Pro-Gln (SEQ ID NO: 28), Lys-Leu-Leu-Glu-Thr-Glu-Cys-Pro-Gln-Tyr (SEQ ID NO: 100), or Lys-Leu-Leu-Glu-Thr-Glu-Cys-Pro-Gln-Tyr-Ile (SEQ ID NO: 101).

The peptide may have a modification on the main chain or side chain. The modification is not limited here unless it interferes with a dentin-forming function of the peptide. The modification may be present in at least one location selected from the N-terminus of the main chain, the C-terminus of the main chain, and at least one side chain of amino acid residues constituting the main chain. The peptide may also be in a cyclic structure.

The N-terminal modification may include those commonly used as N-terminal modifications of peptides. For example, the N-terminal modifications may include azidation, C₁₋₆ acylation (acetylation, formylation, etc.) such as C₁₋₆ alkanoyl, succinylation, Boc modification, Fmoc modification, biotinylation, myristoylation, palmitoylation, stearoylation, fluorescent dye modification (TAMRA labeling, FITC labeling, rhodamine labeling, FAM labeling, etc.), pyroglutamylation, dansylation, methylation, and the like.

The C-terminal modification may include those commonly used as C-terminal modifications of peptides. For example, the C-terminal modifications may include amidation, the biotinylation, methyl esterification, aldehydation, N-hydroxyesterification, pNA labeling, MCA labeling, β naphthylamidation, and the like.

The modification of the side chain of the amino acid residues constituting the main chain may include polyethylene glycolation, phosphorylation, the acetylation, the methylation, fluorescence modification, the biotinylation, modification with sugar or sugar chains, lipid modification, and the like.

It is preferred that the peptide functions to promote formation of dentin in vivo. It is also preferred that the peptide forms denser dentin than when S100-A8 recombinant protein is used. Here, the dentin is preferably tertiary dentin.

Whether or not the formation of the dentin is promoted, or the quality of the formed dentin, may be evaluated, for example, by microscopic examination using an HE-stained sample or by image interpretation using micro-CT on a tooth subjected to a direct pulp capping experiment described in the examples below.

For an evaluation using HE staining or the micro-CT to determine whether or not the dentin formation is promoted, for example, the method described in Clinical Oral Investigations 2018, 22(8):2879-2887.; Journal of Dentistry 2010, 38(10):828-837 may be used to score and quantify the tertiary dentin that has been formed. In this method, a tertiary dentin formation in the area less than one-third of an exposed pulp region can be scored as Grade 0, a tertiary dentin formation in the area of one-third to two-thirds of the exposed pulp region can be scored as Grade 1, a tertiary dentin formation in the area more than two-thirds of the exposed pulp region can be scored as Grade 2, or a tertiary dentin formation in the area completely covering the exposed pulp region can be scored as Grade 3.

### 2. Composition

A composition comprises the peptide described in the above section 1, a pharmaceutically acceptable carrier, and a supporting material.

Examples of the supporting material may include Mineral Trioxide Aggregate (MTA), a paste containing a polymer and hydroxyapatite, gelatin, collagen, and the like. The amount of the peptide added to the supporting material is from about 10 mg to 100 mg per gram of the supporting material.

The composition may comprise the peptide so that the amount of the peptide used per unit volume is about 0.1 to 100 mg/mm³.

The composition may be used to promote the dentin formation. The composition may be used as a composition for promoting pulp wound healing. The composition may also be used as a composition for filling a tooth cavity.

### Examples

The present invention will be described in more detail below with reference to examples. However, the present invention should not be construed as being limited to the examples.

### I. Example 1: Investigation of effect of MMP-20 on S100-A7 during process of tertiary dentin formation

In order to determine whether the full length of the S100-A7 or a degradation product of the S100-A7 degraded by the MMP-20 is required for the pulp wound healing process, the following experiment was performed.

### 1. Materials and Methods

### 1) Direct pulp capping experiment using S100-A7 and S100-A7 reacted with MMP20

This study was conducted under the approval of the Animal Experiment Committee of the Graduate School of Dentistry, Osaka University (approval number: 28-013-0).

Eight-week-old male Wistar rats were injected intraperitoneally with pentobarbital sodium (SomnoPentyl(Trade Mark), Kyoritsu Seiyaku Corporation, Tokyo) (30 mg/kg) as general anesthesia, and carprofen (Rimadyl^{®}, Pfizer, USA) (3 mg/kg) was administered for pain relief. The tooth to be treated was isolated by rubber dam dry field technique, and a surface and surrounding tissue of the tooth were cleaned with an alcohol-soaked cotton ball, and then a cavity was formed using the following method based on the report by Komichi et al. [Non Patent Literature 1]. An experimental cavity was formed in the left and right maxillary first molars using a round bar (#1, Dentsply Maillefer, Switzerland) attached to an electric engine (VIVAMATE G5 ^{®}, NSK, Tochigi) to expose the pulp from an occlusal surface to a mesial pulp horn. After washing the surface of the pulp with saline (Otsuka Pharmaceutical, Tokyo) and confirming hemostasis, direct pulp capping was performed using a gelatin sponge (Spongel ^{®}, Astellas Pharma Inc., Tokyo) soaked with 20 µl of either Dulbecco-phosphate buffered saline (PBS, NACALAI TESQUE, INC., Kyoto) containing the S100-A7 (PROSPEC, Israel) at a concentration of 1 µg/ml or the Dulbecco-phosphate buffered saline containing the S100-A7 treated with the MMP-20 (Funakoshi Co., Ltd., Tokyo) (0.01 µg/ml MMP-20 was added and left at 37 °C for 24 hours). The cavities were then filled with glass ionomer cement (Fuji IX^{™}, GC, Tokyo). Four weeks after the pulp capping, the experimental animals were sacrificed by intraperitoneal injection of an excess of pentobarbital sodium and perfusion fixation with 4 % paraformaldehyde phosphate buffer solution (NACALAI TESQUE, INC., Kyoto) was performed. Maxillas, including the test teeth, were then resected and soft tissues were removed, and evaluated by the following methods. The number of samples was 3 for each condition.

### 2) Image analysis using micro-CT

Image analysis using the micro-CT (R-mCT2^{™}, Rigaku Corporation, Tokyo) was performed for quantitative evaluation of the formed tertiary dentin in the samples after the direct pulp capping. Tomography of the test teeth was performed under the conditions of tube voltage of 90 kV, tube current of 160 µA, and slice width of 5 µm. The horizontal section of the maxillary first molar was defined as the XY plane, the sagittal section of the maxillary first molar was defined as the YZ plane, and the frontal section of the maxillary first molar was defined as the ZX plane, and the opaque image seen in the YZ plane just below the exposed pulp region was defined as the newly formed tertiary dentin. A CT value equivalent to that of the primary dentin was set as a threshold for extracting the tertiary dentin, and the area of the newly formed tertiary dentin was calculated from a binarized image. The area of the newly formed tertiary dentin was measured in each section, and the volume of the newly formed tertiary dentin was calculated by summing all of the measurements. TRI 3D-BON (Ratoc System Engineering Co., Ltd., Tokyo) was used as an image analysis software.

### 3) Histopathological evaluation

The samples used for the micro-CT analysis were immersion-fixed in 4% paraformaldehyde phosphate buffer solution for 12 hours at 4 °C, and then demineralized in Kalkitox (FUJIFILM Wako Pure Chemical Corporation, Osaka) for 2 days at low temperature. After demineralization, the samples were dehydrated in an ascending alcohol series, paraffinembedded, and sectioned serially with a rotary microtome (RM 2155, Leica, Germany) at a thickness of 5 µm, followed by hematoxylin-eosin (HE) staining. Histopathological evaluation of the tertiary dentin was performed under a light microscope (BZ-X700, KEYENCE Corporation, Osaka).

### 4) Statistical analysis

Comparison of the volume of the tertiary dentin measured by the image analysis after the micro-CT imaging was performed using Student's t-test for statistical significance test with a critical value of 5 %.

### 2. Results

As a result of the micro-CT analysis, the tertiary dentin formation completely covering the exposed region of pulp was observed in both cases where the S100-A7 alone was used as a pulp capping material and where the S100-A7 reacted with the MMP20 was used as the pulp capping material (FIG. 1A).

As a result of quantification and comparison of the volume of the formed tertiary dentin, no significant differences were found between the respective samples (p > 0.05) (FIG. 1B).

From an image of an HE-stained sample in which closure of the surface of the exposed pulp was observed by the micro-CT analysis, it was showed that although there were small defects in the tertiary dentin in both samples, there were no large defects where the pulp and tooth crown sides were communicated, and it was confirmed that the surface of the exposed pulp was completely closed by the formed tertiary dentin (FIG. 2).

### II. Example 2: Search for functional peptide derived from Protein S100 family

The fact that the pulp wound healing was promoted despite the direct pulp capping experiment in the rat using the human-derived Protein S100 suggests that the amino acid sequence derived from the Protein S100, which is conserved in different animal species, is likely to function. Therefore, the following experiments were conducted to search for functional sequences conserved among the Protein S100 family.

### 1. Materials and Methods

### 1) Search for amino acid sequence homology in Protein S100 family using sequence alignment

Sequence alignment was performed to search for regions with high homology in the amino acid sequences of the three types of proteins, S100-A7, S100-A8, and S100-A9, whose presence in Dentin Matrix Components (DMCs) has been reported and whose involvement in the pulp wound healing has been suggested, in 8 mammalian sequences of Polar bear, Brandt's bat, Chinese tree shrew, Black flying fox, David's myotis, Human, Bovine, and Horse for the S 100-A7, 4 mammalian sequences of Human, Bovine, Mouse, and Rat for the S 100-A8, and 5 mammalian sequences of Human, Bovine, Rabbit, Mouse, and Rat for the S 100-A9. Amino acid sequences used for comparative study were those registered in Uniprot (https://www.uniprot.org/), which is a comprehensive database of protein amino acid sequences, and the sequence alignments were performed using CLUSTAL-W (https://www.genome.jp/tools-bin/clustalw), which is a multiple alignment program.

### 2) Screening of functional peptides using peptide array technique

Candidate peptides for use in peptide array technique, a screening method for the functional peptides, were selected from the amino acid sequence of the human Protein S100 from regions with high homology in the amino acid sequences of S100-A7, S100-A8, and S 100-A9 obtained by the sequence alignment. In order to determine the number of amino acid residues of the peptide, we decided to use 9 residues in this study, based on the following information: about 80% of more than 100 peptide drugs currently existing are peptide drugs with 10 residues or less, and W9 peptide, which has been reported to bind to Receptor Activator of Nuclear factor-Kappa B Ligand (RANKL) on osteoblasts and promote osteogenic signaling, consists of 9 residues, etc.

For the purpose of screening the function of the candidate peptides selected based on the condition described above, the peptide array technique was performed based on the report by Kanie et al [Materials 2016, 9(9):730]. The peptides were synthesized on cellulose membranes by Fmoc solid-phase synthesis technique using a peptide synthesizer (ASP222, Intavis, Germany) and placed in a 96 well plate. Human pulp stem cells (hDPSCs, Lonza, Switzerland) were seeded in the same plate at 5,000 cells/well and cultured in mineralization induction medium (50 µg/ml ascorbic acid (Sigma Aldrich, USA), 10 mM β-glycerophosphate (Sigma Aldrich), and Minimum Essential Medium Eagle (Alpha Modification) (α-MEM) with 10% FBS) at 37 °C in a 5% CO₂ gas phase for 14 days. The medium was replaced every 3 days. After 14 days from the start of culture, the medium was removed, washed with the PBS, fixed in 10 % neutral buffered formalin (FUJIFILM Wako Pure Chemicals Corporation, Osaka) for 30 minutes, stained with alizarin red solution for 30 minutes using the mineralization assay kit (PG Research, Tokyo), washed with purified water, added with mineralized nodule lysate from the same kit, stirred for 10 minutes, and supernatant containing eluted dye was used for quantitative evaluation of mineralization ability of the cells. The evaluation was performed by measuring absorbance at 450 nm using a microplate reader (ARVO MX, PerkinElmer, USA). The number of samples was 9 for each condition. A random peptide (amino acid sequence is KHHAKVGSA: SEQ ID NO: 59, also referred to as "No. 59 peptide"), which is known to have no ability to induce mineralization, was used as a negative control, and Bone morphogenetic protein (BMP)-derived peptide (amino acid sequence is TLVNSVNSK: SEQ ID NO: 60, also referred to as "No. 60 peptide") which has been reported to have osteogenic ability and also promotes pulp wound healing, was used as a positive control.

### 2. Results

### 1) Search for amino acid sequence homology in Protein S100 family using sequence alignment

The sequence alignment was performed to search for regions with high homology in the amino acid sequences of the three types of proteins, S100-A7, S100-A8, and S100-A9, in a plurality of mammalian species. As a result, three regions with high homology were found in shaded regions (A, B, and C in FIG. 3), and candidate peptides were selected from these regions for the peptide array technique to be used subsequently. As shown in FIG. 4, 9 residues from the N-terminus of the amino acid sequence of the human Protein S100 were selected as the candidate peptide from the region with high homology in the sequence alignment (FIG. 3). Those shifted by three residues from the candidate peptide were also considered as the candidate peptides, and a total of 58 candidate peptides were selected from the regions A, B, and C with high homology for the S100-A7, S100-A8, and S100-A9 (FIG. 5).

### 2) Screening of functional peptides using peptide array technique

The quantitative results of the mineralization ability of the hDPSCs in the presence of the 58 candidate peptides are shown in FIG. 6. The top 10 peptides inducing the mineralization of the hDPSCs are indicated by "*" in FIG. 6, and the results of comparing these 10 peptides with the results of the region selected for peptide in the sequence alignment are shown in FIG. 7. In two black boxed areas in FIG 7, many peptides that promoted mineralization of hDPSCs overlapped. Based on the overlapped regions, four peptides, that is, a peptide derived from the S100-A8 (amino acid sequence is KLLETECPQ: SEQ ID NO: 28, also referred to as "No. 28 peptide") and a peptide derived from the S100-A9 (amino acid sequence is ELVRKDLQN: SEQ ID NO: 47, "No. 47 peptide") from the region in FIG. 7-b, and a peptide derived from the S100-A8 (amino acid sequence is NTDGAVNFQ: SEQ ID NO: 32, also referred to as "No. 32 peptide") and a peptide derived from the S100-A9 (amino acid sequence is NADKQLSFE: SEQ ID NO: 51, also referred to as "No. 51 peptide") from the region in FIG. 7-c, were considered likely to be functional sequences, and verified in the following experiments.

### III. Example 3: Investigation of effect of Protein S100 family-derived peptide on pulp wound healing

The following experiments were performed to investigate the effects of No. 28 peptide, No. 47 peptide, No. 32 peptide, and No. 51 peptide, which are the functional peptides derived from the Protein S100 family, on pulp wound healing.

### 1. Materials and Methods

### 1) Evaluation of effects of Protein S100 family-derived peptide on toxicity to hDPSCs and proliferative capacity of hDPSCs

The hDPSCs were seeded in α-MEM medium containing 10 % FBS added with the four peptides at concentrations of 0.1, 1, 10, and 100 µg/ml in a 96 well plate at 5,000 cells/well and cultured at 37 °C for 2 days in a 5 % CO₂ gas phase. For toxicity test, culture supernatant was collected and LDH-assay was performed using LDH Cytotoxicity Detection Kit (Takara Bio Inc., Shiga). A reagent was added to the collected culture supernatant, and the culture supernatant was placed at 37 °C for 30 minutes in the 5 % CO₂ gas phase. The absorbance at 405 nm was then measured using the microplate reader (ARVO MX). To further evaluate the proliferative capacity, WST-1-assay was performed using Premix WST-1 Cell Proliferation Assay System (Takara Bio Inc.) after the cells had been continuously cultured for 1 day. The culture was added with a WST-1 reagent and allowed to stand for 30 minutes at 37 °C in the 5% CO₂ gas phase. After shaking for 1 minute at room temperature, the absorbance at 450 nm was measured using the microplate reader (ARVO MX). A sample in which the PBS was added to the culture was used as a control, and the number of samples was 6 for each condition.

### 2) Direct pulp capping experiment using Protein S100 family-derived peptide

To evaluate the effect of the Protein S100 family-derived peptide on ability of forming of the tertiary dentin, a direct pulp capping experiment was performed on rat molars using a gelatin sponge (Spongel ^{®} ) soaked with 20 µl of each PBS solution containing the No. 28, No. 47, No. 32, or No. 51 peptide at a concentration of 100 µg/ml using the same way as described in the section 1) of "Materials and Methods" of the section 1 of the section I above. PBS solution containing the S100-A8 (PRO SPEC, Israel), the PBS solution containing the S100-A9 (PROSPEC), wherein the S100-A8 and S100-A9 are a recombinant of the protein from which the peptide was derived, at a concentration of 1 µg/ml, and the PBS solution alone were used as controls. After 4 weeks from the capping treatment, the micro-CT imaging and image analysis were performed on the tertiary dentin formed just below the exposed pulp. An evaluation method, in which the amount of formed tertiary dentin observed in histopathological images was scored, in the direct pulp capping experiment conducted by Okamoto et al. in the past, was applied to the micro-CT images in this study, and the evaluation was performed by scoring the formed tertiary dentin [Clinical Oral Investigations 2018, 22(8):2879-2887.; Journal of Dentistry 2010, 38(10):828-837]. The evaluation was performed by one person, with a score of Grade 0 in cases where the tertiary dentin was formed in only one-third or less of the exposed pulp region, Grade 1 in cases where the tertiary dentin was formed in one-third to two-thirds of the exposed pulp region, Grade 2 in cases where the tertiary dentin was formed in two-thirds or more of the exposed pulp region, and Grade 3 in cases where the formed tertiary dentin was completely covering the exposed pulp region. In addition, the same micro-CT images were analyzed using the same way as described in the section 2) of "Materials and Methods" of the section 1 of the section I above to evaluate the volume of the formed tertiary dentin. HE staining was also performed using the same way as described in the section 3) of "Materials and Methods" of the section 1 of the section I above, and histopathological evaluation of the formation of the tertiary dentin was performed. The number of samples was 8 for each condition.

### 3) Statistical analysis

The statistical significance test in the above experiment was performed using the Oneway ANOVA and Tukey's test for a cytotoxicity test and Kruskal-Wallis-test for a cell proliferation test and a comparison of the amount of formed tertiary dentin in the direct pulp capping experiment, with a critical value of 5 %.

### 2. Resits

### 1) Evaluation of effect of Protein S100 family-derived peptide on toxicity to hDPSCs and proliferative capacity of hDPSCs

Cytotoxicity of each peptide was assessed by the LDH-assay. As a result, compared to the control, significantly lower cytotoxicity was observed in samples with all concentrations of No. 28 peptide and No. 47 peptide, 1 µg/ml of No. 32 peptide, and 100 µg/ml and 10 µg/ml of No. 51 peptide (p < 0.05) (Fig. 8A). No significant difference was observed between the other samples and the control (p > 0.05).

On the other hand, there was no significant difference in the effect of each peptide on the proliferative capacity of hDPSCs between all samples and the control (p>0.05) (FIG. 8B).

These results showed that all peptides (No. 28 peptide, No. 47 peptide, No. 32 peptide, and No. 51 peptide) exhibited the cytotoxicity comparable to that of the PBS and had no effect on cell proliferation. Based on these results, the direct pulp capping experiment was conducted in rats using 100 µg/ml of the peptides to examine the function of each peptide in vivo.

### 2) Direct pulp capping experiment using Protein S100 family-derived peptide

FIG. 9A shows results of scoring the tertiary dentin formed after the pulp capping. The results of the pulp capping with the No. 28 peptide showed the tertiary dentin formation that completely sealed the exposed pulp region in 5 samples, while the results of the pulp capping with the other peptides showed the formation of the tertiary dentin that completely sealed the exposed pulp region in 0 or only 1 sample. The tertiary dentin formation that completely sealed the exposed pulp region was observed in 1 sample treated with the recombinant protein S100-A8, 2 samples treated with the S100-A9, and 0 samples treated with the PBS, as controls.

As a result of quantifying the volume of the tertiary dentin, it was found that the volume of the tertiary dentin was significantly higher in the sample subjected to the pulp capping with No. 28 peptide than in the sample subjected to the pulp capping with No. 47 peptide or the PBS (p < 0.05), but there was no significant difference from the other samples (p > 0.05) (FIG. 9B).

FIG. 10 shows representative results of the HE staining of the samples used for the micro-CT analysis. In the samples treated with the No. 28 peptide, No. 32 peptide or No. 51 peptide in which complete tertiary dentin formation was observed, it was showed that although there were small defects in the tertiary dentin, there were no defects where the pulp and tooth crown sides were communicated, and it was confirmed that the surface of the exposed pulp was completely closed by the formed tertiary dentin. In the samples treated with the S100-A8 or S100-A9, no defects were observed where the pulp and tooth crown sides were communicated, and it was confirmed that the surface of the exposed pulp was completely closed by the formed tertiary dentin, but the tertiary dentin showed a sparse structure compared to the samples treated with the peptides (FIG. 10).

The present study revealed that the functional peptide (KLLETECPQ: SEQ ID NO: 28) derived from the Protein S100-A8, which is present in a dentin matrix protein, promotes the pulp wound healing the most.

### IV. Example 4: Analysis of mechanism of pulp wound healing by Protein S100 family-derived peptide

To investigate the mechanism by which the Protein S100 family-derived peptide, which showed good results in the direct pulp capping experiment, promoted the pulp wound healing, the interaction between the No. 28 peptide and pulp cells was induced, and the changes in the proteins expressed by the pulp cells were analyzed by liquid chromatography-tandem mass spectrometry (LC-MS/MS).

### 1. Materials and Methods

### Proteome analysis of pulp wound healing process caused by Protein S100 family-derived peptide

The No. 28 peptide or the recombinant S100-A8, the protein from which the No. 28 peptide is derived, was applied to the hDPSCs to compare and quantify the proteins expressed by the hDPSCs by the LC-MS/MS. The hDPSCs were seeded in a 100 mm cell culture dish at 3,000,000 cells/plate in the mineralization induction medium containing each of the samples containing the No. 28 peptide or the S100-A8 at a concentration of 0.1 mM, and cultured at 37 °C in the 5% CO₂ gas phase for 7 days. Then, plasma membrane and cytoplasmic proteins were extracted using a plasma membrane protein extraction kit (Cosmo Bio Co., Ltd., Tokyo). As a practical procedure, the hDPSCs were cultured for 7 days, collected with a scraper, added with Buffer A included in the kit, and left on ice for 10 minutes. The hDPSCs were then transferred into a microtube with a filter cartridge included in the kit and centrifuged at 14,000 rpm for 30 seconds. After discarding the cartridge and agitating for 10 seconds, the microtube was centrifuged at 3000 rpm for 1 minute and the supernatant was collected. The collected supernatant was then centrifuged at 14,000 rpm for 30 minutes to extract the cytoplasmic proteins separated in a supernatant and the plasma membrane proteins separated in pellet. As a control, the hDPSCs cultured in mineralization induction medium supplemented with the PBS were used.

Each sample was analyzed by liquid chromatography using UltiMate 3000 Nano LC systems (Thermo Fisher Scientific, USA) and ESI-column (0.075 x 150 mm) (Thermo Fisher Scientific). A 0.1 % formic acid aqueous solution containing acetonitrile was used as a mobile phase at a flow rate of 300 nL/min. The concentration of acetonitrile was 5 % for the first 5 min, increased to 40 % in 95 min, then increased at a constant rate for 2 min to 90 %, and maintained for 5 min. The peptides were analyzed using Q-Exactive (Thermo Fisher Scientific). Identification and quantitative analysis of the proteins were performed by peptide mass fingerprinting using Mascot Distiller v2.5, Mascot Server v2.5 (Matrix Science, USA), and UniProt on obtained MS/MS spectra.

In addition, to evaluate changes in expression levels of the proteins, the following criteria were used to narrow down the identified plasma membrane-derived and cytoplasm-derived proteins among the samples.

### <Criteria for narrowing down proteins whose expression levels increased in peptide group and S 100-A8 group compared to control group>

- Proteins whose measured values were higher in the S 100-A8 group than in the control group
- Proteins whose measured values in the peptide group are more than twice as high as those in the control group
- Proteins whose measured values in both the peptide group and the S100-A8 group are equal to or greater than 1 (a.u.; arbitrary unit)

### <Criteria for narrowing down proteins whose expression levels decreased in peptide group and S 100-A8 group compared to control group>

- Proteins whose measured values were lower in the S 100-A8 group than in the control group
- Proteins whose measured values in the control group are more than twice as high as those in the peptide group
- Proteins whose measured values in the control group are equal to or greater than 1 (a.u.; arbitrary unit)

Each of the groups narrowed down by the above criteria was analyzed using Gene Ontology (GO) classification. The GO provides an internationally standardized GO Term, which is intended to describe biological functions. The GO Term is organized in a hierarchical structure, with the upper order representing general functions and the lower order representing more detailed functions. For example, there are three categories at the top: "Molecular function," "Biological process," and "Cellular component."

The GO Term belonging to "Molecular function" and "Biological process" which represent their respective functions from the above classification were searched for the protein groups obtained in this experiment, and the functions characteristic to each protein group were examined.

### 2. Results

### Proteome analysis of pulp wound healing process caused by Protein S100 family-derived peptide

As a result of the LC-MS/MS analysis, 1833 plasma membrane-derived proteins and 1557 cytoplasm-derived proteins were identified.

As a result of examining the proteins whose expression levels changed according to each narrowing down criteria, in the plasma membrane-derived proteins, 158 proteins whose expression levels were more increased in the sample treated with the No. 28 peptide or S100-A8 than in the sample treated with the control were detected, while 97 proteins whose expression levels were more decreased in the sample treated with the No. 28 peptide or S100-A8 than in the sample treated with the control were detected. Similarly, in the cytoplasm-derived proteins, 250 proteins whose expression levels were more increased in the sample treated with the No. 28 peptide or S100-A8 than in the sample treated with the control were detected, while 67 proteins whose expression levels were more decreased in the sample treated with the No. 28 peptide or S100-A8 than in the sample treated with the control were detected.

Next, for the narrowed down protein groups, the GO Term of each protein was searched and the characteristic functions of each protein were examined, and the following results were obtained (FIG. 11). First, for the proteins whose expression levels were more increased in the sample treated with the No. 28 peptide or S100-A8 than in the sample treated with the control, proteins with the GO Term related to a function of promoting Nuclear Factor-kappa B (NFκB) signaling pathway were characteristically found. Specifically, Flotillin-1 (FLOT1), Very-long-chain (3R)-3-hydroxyacyl-CoA dehydratase (3KCR1), Protein LYRIC (LYRIC), and Gap junction alpha-1 protein (GJA1) were obtained from the plasma membrane-derived proteins, and Protein S100A-13 (S100A-13) and Nucleolar protein 3 (NOL3) were obtained from the cytoplasm-derived proteins. On the other hand, for the proteins whose expression levels were decreased in the sample treated with the No. 28 peptide or S100-A8 than in the sample treated with the control, proteins with the GO Term related to a function of promoting Mitogen-activated Protein Kinase (MAPK) signaling pathway were characteristically found. Specifically, Dual specificity mitogen-activated protein kinase kinase 1 (MAP2K1), Cadherin-2 (CDH2), GTPase Nras (NRAS), and Mitogen-activated protein kinase 1 (MAP1) were obtained from the plasma membrane-derived proteins, and Adapter molecule crk (CRK) and Proteasome subunit beta type-7 (PSMB7) were obtained from the cytoplasm-derived proteins.

These results suggest that the No. 28 peptide may promote the mineralization of the DPSCs by suppressing expression of proteins related to the MAPK signaling pathway and promoting expression of proteins related to the NFκB signaling pathway. It has been reported that the NFκB signaling pathway generally regulates immunity, inflammation, cell proliferation, and cell differentiation, while it has been reported that the NFκB signaling pathway was activated in mouse odontoblast-like cells in which Neurotrophin receptor-interacting MAGE homologue (Nrage) was knocked down and the activity of ALP increased, and that the NFκB signaling pathway was activated by applying H₂O₂ to pulp cells overexpressing Peroxisome proliferator-activated receptor gamma (PPARy), thereby promoting mineralization. It has also been reported that the MAPK signaling pathway generally regulates cell proliferation, cell differentiation, cell migration, and apoptosis, and that an administration of 5-Methoxytryptophan (5MTP) to mouse suppresses the MAPK pathway and exerts anti-inflammatory effects in a neural tissue after spinal cord injury. In this experiment, the expression of proteins that activate the MAPK pathway was decreased in the samples treated with the No. 28 peptide, suggesting that the suppression of inflammation may promote the healing.

### Sequence Listing

P22-087WO.XML

## Claims

1. A peptide comprising
an amino acid sequence comprising Lys-Leu-Leu-Glu-Thr-Glu-Cys-Pro-Gln (SEQ ID NO: 28);
an amino acid sequence comprising Asn-Thr-Asp-Gly-Ala-Val-Asn-Phe-Gln (SEQ ID NO: 32);
an amino acid sequence comprising Glu-Leu-Val-Arg-Lys-Asp-Leu-Gln-Asn (SEQ ID NO: 47);
an amino acid sequence comprising Asn-Ala-Asp-Lys-Gln-Leu-Ser-Phe-Glu (SEQ ID NO: 51);
an amino acid sequence having such a structure that 1 to 3 amino acid residues in the amino acid sequence represented by SEQ ID NO: 28, SEQ ID NO: 32, SEQ ID NO: 47 or SEQ ID NO: 51 are substituted or deleted; or
an amino acid sequence having such a structure that 1 to 3 amino acid residues are inserted into the amino acid sequence represented by SEQ ID NO: 28, SEQ ID NO: 32, SEQ ID NO: 47 or SEQ ID NO: 51,
the peptide having a length of 9 to 15 amino acid residues.

2. The peptide according to claim 1, wherein the peptide comprises the amino acid sequence comprising
Lys-Leu-Leu-Glu-Thr-Glu-Cys-Pro-Gln (SEQ ID NO: 28),
Lys-Leu-Leu-Glu-Thr-Glu-Cys-Pro-Gln-Tyr (SEQ ID NO: 100), or
Lys-Leu-Leu-Glu-Thr-Glu-Cys-Pro-Gln-Tyr-Ile (SEQ ID NO: 101).

3. A composition comprising the peptide according to claim 1 or 2.

4. The composition according to claim 3, wherein the composition is used to promote the formation of dentin.

5. The composition according to claim 3, wherein the composition is for filling a tooth cavity or for promoting pulp wound healing.
